# FASCICULE DE BREVET EUROPEEN

(11) **EP 2 563 376 B1**
(45) Date de publication et mention de la délivrance du brevet: **22.11.2017**
(21) Numéro de dépôt: 11721344.7
(22) Date de dépôt: 22.04.2011
(51) Int. Cl.: A61K 8/92, A61K 9/00, A61K 45/06, A61K 47/14, A61K 9/06, A61K 31/232, A61K 36/185, A61Q 19/00, A61P 17/00, A61K 8/73, A61K 8/97, A61K 31/728

(54) **COMPOSITION DERMATOLOGIQUE À BASE D'HUILE D'ARGAN ET D'ACIDE HYALURONIQUE**
DERMATOLOGISCHE ZUSAMMENSETZUNG ENTHALTEND ARGAN ÖL UND HYALURONSÄURE
DERMATOLOGICAL COMPOSITIONS COMPRISING ARGAN OIL AND HYALURONIC ACID

(30) Priorité: 30.04.2010 FR 1001849
(43) Date de publication de la demande: 06.03.2013
(73) Titulaire: Ayawane, 69100 Villeurbanne (FR)
(72) Inventeur: DAHRI CORREIA, Latifa, F-38080 l'Isle d'Abeau (FR)
(74) Mandataire: Schmidt, Martin Peter
(86) Numéro de dépôt international: PCT/FR2011/000245
(87) Numéro de publication internationale: WO 2011/135200

(56) Documents cités:
- EP-A1- 2 133 066
- WO-A1-02/09728
- WO-A1-2009/125182
- DE-A1-102008 044 700
- FR-A1- 2 756 183
- US-A1- 2008 199 489
- US-A1- 2010 098 794

## Description

### Domaine de l'invention

L'invention appartient au domaine des compositions cosmétiques et/ou pharmaceutiques à base de composés naturels, et possédant des activités biologiques cicatrisantes, réparatrices des traumatismes cutanés et anti-inflammatoires, pour une utilisation prophylactique ou curative par voie topique.

### Etat de la technique

L'huile d'argan est extraite des noyaux des baies d'arganier (Argania spinosa L.), espèce végétale endémique du sud-ouest marocain. Comme décrit dans le livre « La pharmacopée marocaine traditionnelle » par Jamal Bellakdar, pages 486 - 487, l'huile d'argan est extraite des noyaux des baies de l'arganier, soit de manière artisanale, soit industriellement par pressage ou par extraction par un solvant apolaire. L'huile d'argan, connue depuis des décennies pour son action hydratante, nutritive et adoucissante de la peau, est utilisée dans de nombreuses préparations en dermatologie pour la peau et les cheveux, mais aussi en rhumatologie, en oto-rhino-laryngologie, contre les spasmes intestinaux, contre l'asthénie. L'huile d'argan contient en particulier des acides gras, des stérols, des tocophérols, ses propriétés particulières seraient également dues à sa teneur en vitamine E, qui agit comme un antioxydant naturel, par piégeage des radicaux libres.

L'acide hyaluronique (HA) est un glycosaminoglycane largement présent dans les tissus conjonctif, épithélial et nerveux. On le trouve en particulier dans l'humeur vitrée et dans le liquide synovial. C'est l'un des principaux composants de la matrice extra-cellulaire. L'acide hyaluronique contribue de façon significative à la prolifération et à la migration des cellules. L'acide hyaluronique est un polymère de disaccharides, eux-mêmes composés d'acide D-glucuronique et de D-N-acétylglucosamine, liés entre eux par des liaisons glycosidiques alternées beta-1,4 et beta-1,3. Les polymères de cette unité récurrente peuvent avoir une taille comprise entre 10² et 10⁴ kDa in vivo, mais il existe également des produits avec des masses moléculaires beaucoup plus grandes. L'acide hyaluronique peut être obtenu industriellement, par exemple par broyage de crêtes de coqs, après broyage, ou par fermentation bactérienne (par exemple avec *Streptococcus zooepidemicus*). Par hydrolyse contrôlée, on obtient un produit avec une masse moléculaire plus faible. L'acide hyaluronique est utilisé depuis de nombreuses années en chirurgie ophtalmique (i.e. transplantation de cornée, glaucome, cataracte). Il est également utilisé pour traiter l'arthrose du genou (ce traitement est appelé « viscosupplémentation »). Enfin, depuis quelques années, l'acide hyaluronique est utilisé en médecine esthétique pour le comblement de rides, et il est utilisé dans les produits cosmétiques en raison de ses propriétés hydratantes.

Un certain nombre de documents ont décrit des compositions dermatologiques qui contiennent l'huile d'argan et l'acide hyaluronique, le plus souvent dans des mélanges complexes.

Le brevet FR 2 756 183 (Pierre Fabre Dermo-Cosmétique) décrit une composition pharmaceutique ou cosmétique complexe renfermant en tant que principe actif au moins une association d'huile d'argan et de peptides d'argan. Cette composition peut est utilisée pour le traitement du vieillissement cutané, en particulier le vieillissement lié à la ménopause. Les inventeurs ont mis en évidence une synergie entre l'huile d'argan et les peptides d'argan. Selon ce document, l'huile d'argan possède des activités anti-vieillissement cutané, et en particulier une activité proliférative (relance de l'activité cellulaire), une activité anti-radicalaire, un effet restructurant et hydratant, et un effet nutritif. Dans un exemple de ce brevet, l'acide hyaluronique est présent dans une composition mentionnée à titre d'exemple pour un masque anti-ride (acide hyaluronique (sel de Na) 0,01 à 0,2% ; huile d'argan enrichie 0,5 - 4% ; peptides d'argan 0,01 - 2%) ; aucun effet spécifique de cet acide n'est mentionné.

La demande de brevet EP 1 920 756 (Syrio Pharma S.P.A.) décrit une matrice à base d'eau pour préparer des préparations cosmétiques sans conservateur comprenant 2 à 40% en poids d'au moins d'un ester végétal de glycérine avec un ou plusieurs acides gras mono- ou polyinsaturés, ayant 18 à 20 atomes de carbone ; 0,5 à 20% en poids d'au moins d'un ester de non-triglycéride formé d'un alcool ayant 12 à 22 atomes de carbone, et d'un ou plusieurs acides gras saturé, mono-insaturé et/ou poly-insaturés ayant 14 à 22 atomes de carbone ; 0,5 à 25% en poids d'au moins d'un polyol de fonctionnalité 2 à 12; au moins 0,012% en poids d' au moins d'un agent séquestrant ; 0,2% à 20% en poids d'au moins d'un agent antioxydant ; 0,0001% à 5% en poids d'au moins une fraction non saponifiable d'huile naturelle et/ou de beurre naturel contenant les fractions stéroliques de la plante naturelle de laquelle elle/il dérive. Un autre objet de cette demande de brevet est une formulation cosmétique comprenant la matrice et des agents cosmétiques actifs. L'huile d'argan est citée parmi les esters végétaux de glycérine. L'acide hyaluronique est cité parmi les agents cosmétiques actifs, en tant qu'agent anti-rides ainsi qu'en tant qu'agent hydratant. Dans la formulation, l'agent actif est présent en une quantité comprise entre 0,5 et 10% en poids, par rapport au poids total de la formulation. Aucun exemple concret pour l'association entre l'huile d'argan et l'acide hyaluronique n'est donné.

De nombreux autres documents décrivent des exemples d'association entre des constituants de l'acide hyaluronique et l'huile d'argan dans des compositions complexes. Ainsi, la demande de brevet FR 2 724 663 (Pierre Fabre Dermo Cosmétique) décrit une association entre l'acide hyaluronique et l'extrait insaponifiable d'huile d'argan. La demande de brevet US 2004/0042996 (Cognis Corp.) mentionne l'utilisation de protéines extraites de l'huile d'argan dans une composition qui peut contenir également de l'acide hyaluronique. Le brevet US 7,105,184 (Cognis France) et les demandes de brevet US 2003/0138394 (Cognis Corp.), WO 2005/039610 (Cognis France), WO 2008/080708 (Henkel) et WO 2006/122685 (Greeleaf srl) mentionnent l'utilisation d'extraits d'Argania Spinosa L. comme principe actif dermatologique en association avec un filmogène pouvant contenir de l'acide hyaluronique ou un de ses sels. Les demandes de brevet EP 1 815 843 (Henkel KGaA), US 2009/0047226 (G.Teckenbrock) et EP 1 938 785 (Henkel AG & Co. KGaA) mentionnent l'utilisation de l'huile d'argan comme apaisant dans des compositions dermatologiques qui peuvent également contenir de l'acide hyaluronique comme apaisant ; aucun exemple n'est donné. Aucun de ces documents n'indique un mécanisme d'action ou font état d'une coopération entre ces deux types de composés. Il en est de même des documents WO 2009/043341 (M.Albrecht), WO 2009/080659 (L'Oreal) et US 2008/199489 (V.Parrinello).

Le problème auquel répond la présente invention est de présenter une nouvelle composition à base d'huile d'argan à efficacité dermatologique améliorée.

Dans la présente invention, l'inventeur a découvert que l'ajout d'un faible quantité d'acide hyaluronique de masse moléculaire ne dépassant pas 400 kDa à l'huile d'argan permet d'élaborer une nouvelle composition à base d'huile d'argan possédant des propriétés cicatrisantes et/ou réparatrices des traumatismes cutanés et/ou anti-inflammatoires améliorées, pour une utilisation prophylactique et/ou curative pour le traitement topique de la peau, grâce à un effet synergétique entre l'huile d'argan et l'acide hyaluronique.

### Objet de l'invention

La présente invention a pour objet une composition pour le traitement topique de la peau, et notamment une composition cosmétique ou dermatologique, caractérisée en ce qu'elle comprend, dans un milieu physiologiquement acceptable:
- 1,0 à 50% d'huile d'argan,
- et 0,05 à 2,5% d'acide hyaluronique de masse moléculaire ne dépassant pas 400 kDa, préférentiellement entre 2,0% et 50% d'huile d'argan et entre 0,1% et 2,5% d'acide hyaluronique, et encore plus préférentiellement entre 4,0 et 20% d'huile d'argan et entre 0,1 et 2,0 % d'acide hyaluronique. Le reste est constitué d'ingrédients dermatologiquement et physiologiquement acceptables pour fabriquer une crème, une pommade, une baume, un gel ou une forme similaire qui peut être appliquée sur la peau et qui y reste pendant le temps nécessaire entre deux applications.

L'acide hyaluronique (n° CAS 9004-61-9) est un acide hyaluronique hydrolysé d'une masse moléculaire ne dépassant pas 400 kDa, et de préférence comprise entre 10 et 300 kDa. Il peut se présenter sous la forme d'un sel physiologiquement acceptable, tel que le sel de sodium. On préfère utiliser un acide hyaluronique présentant une masse moléculaire comprise entre 10 et 100 kDa, et encore plus préférentiellement entre 20 et 50 kDa.

La composition selon l'invention comporte avantageusement uniquement ces deux principes actifs dermatologiques, mais on peut ajouter d'autres principes actifs sélectionnes dans le groupe constitué par les anti-biotiques, les anti-septiques, les antimycosiques, les anti-inflammatoires, les antalgiques à application topique, les antalgiques à effet systémique. Elle se présente avantageusement sous la forme d'une crème, pommade, baume ou d'un gel.

La présente invention a également pour objet un procédé pour la fabrication d'une telle composition, dans lequel on prépare d'abord, à une température supérieure à 45°C (de préférence comprise entre à 65°C et 85°C) et sous agitation, une émulsion de type « huile dans l'eau », ladite phase huileuse comprenant l'huile d'argan, puis on laisse refroidir, de préférence à une température ne dépassant pas 30°C et de préférence ne dépassant pas 25°C, et on ajoute à ladite émulsion une phase aqueuse comprenant l'acide hyaluronique ou un de ses sels physiologiquement acceptables.

L'invention vise également l'utilisation de ladite composition pour le traitement de plaies cutanées ou de l'irritation cutanée chez les mammifères.

### Figures

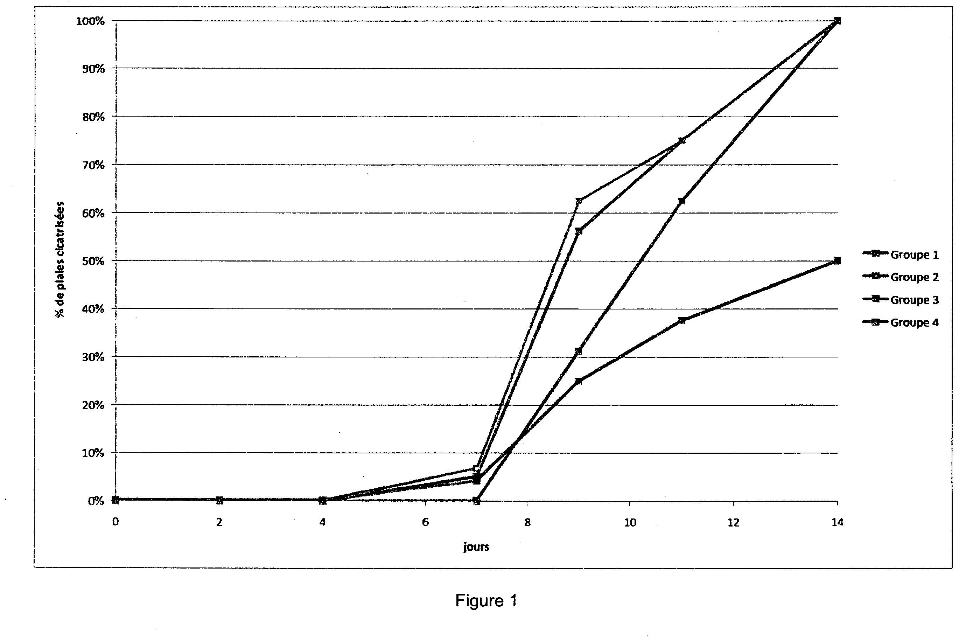

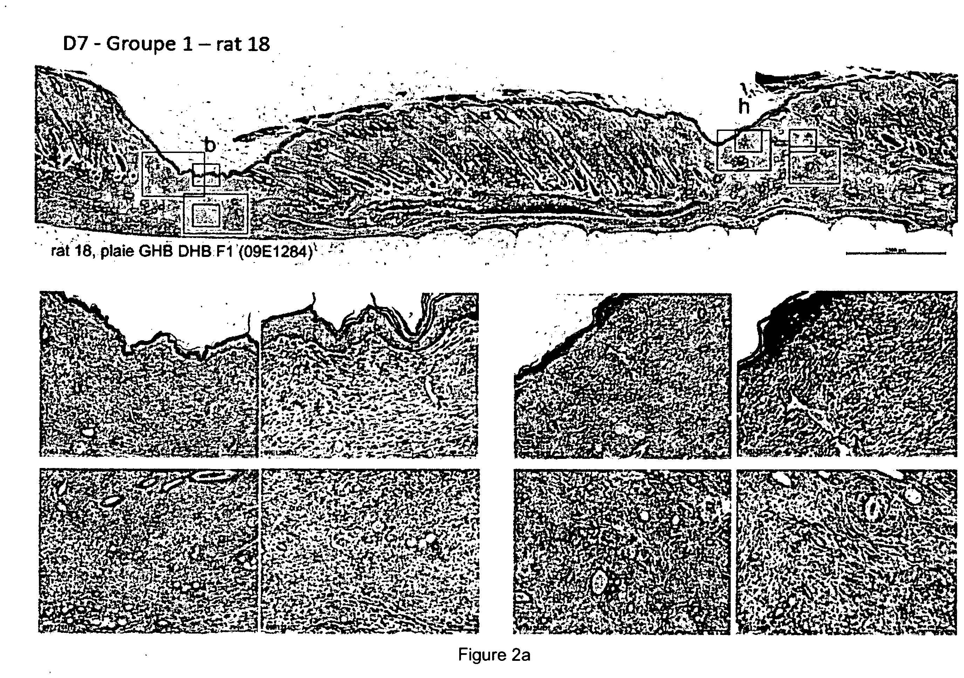

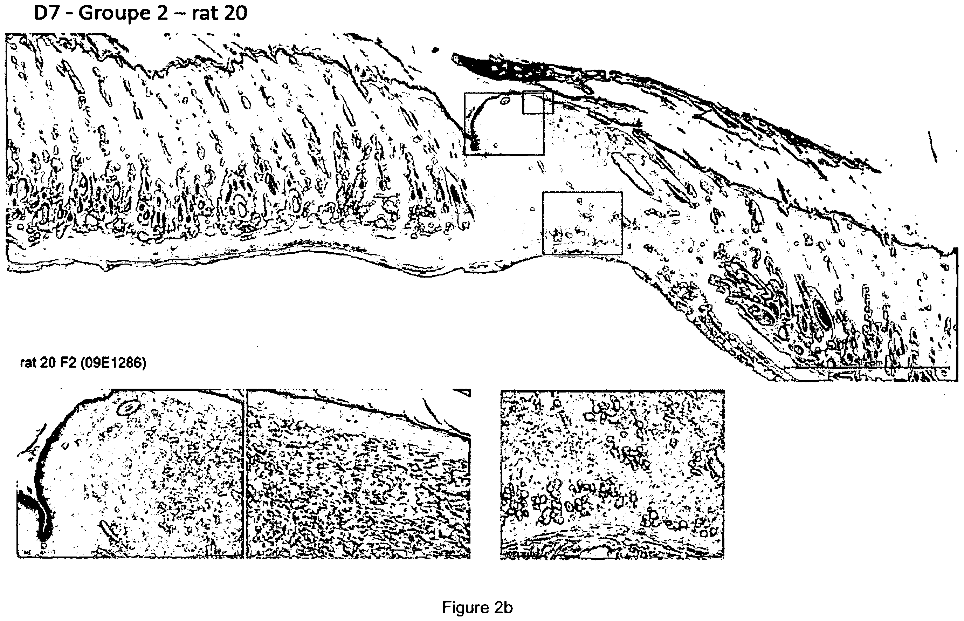

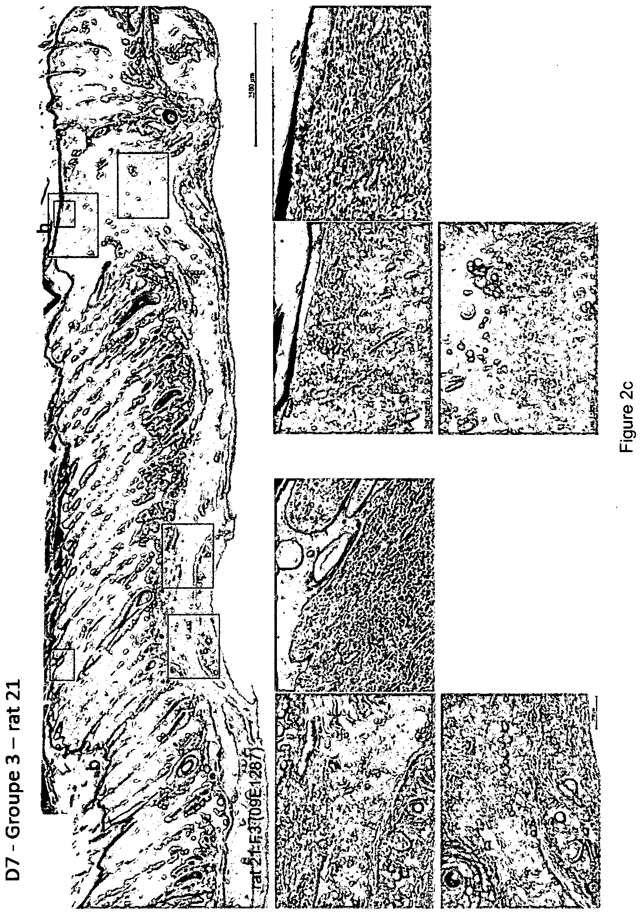

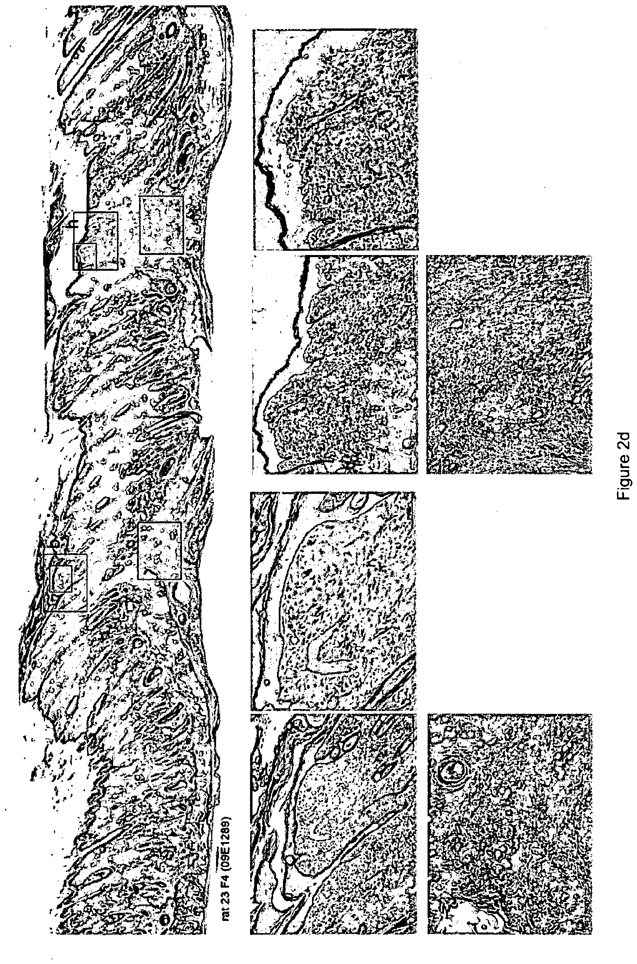

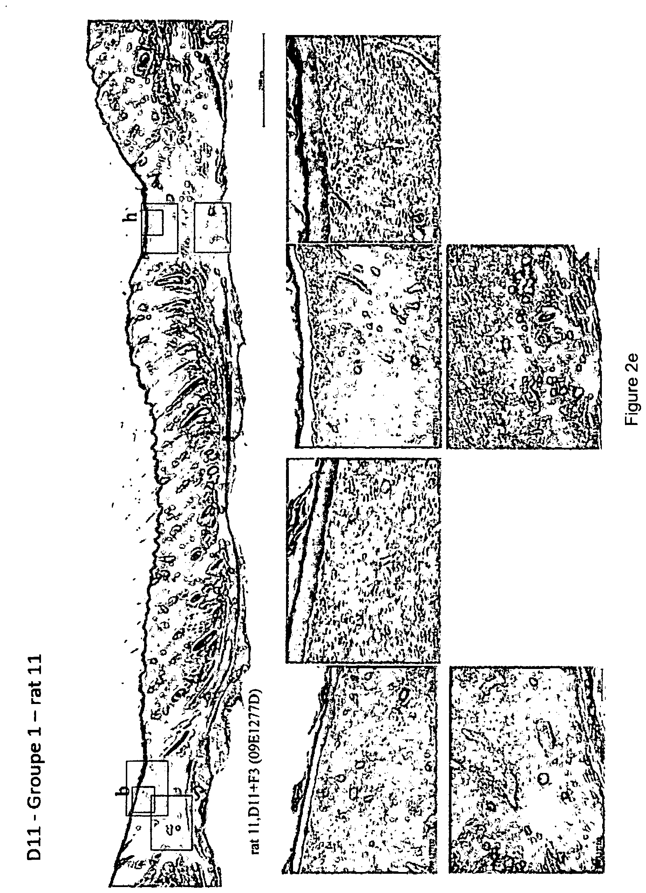

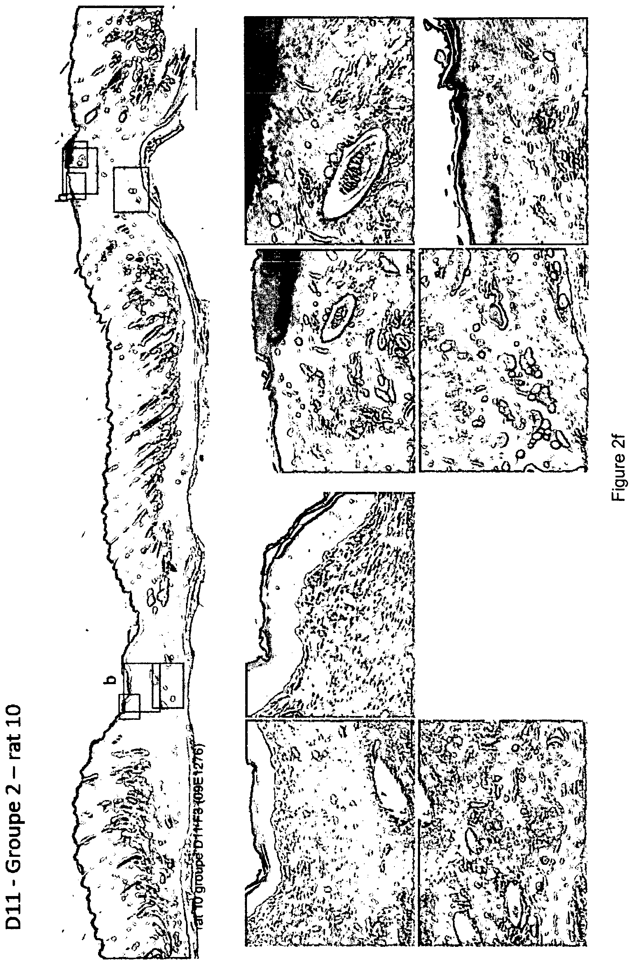

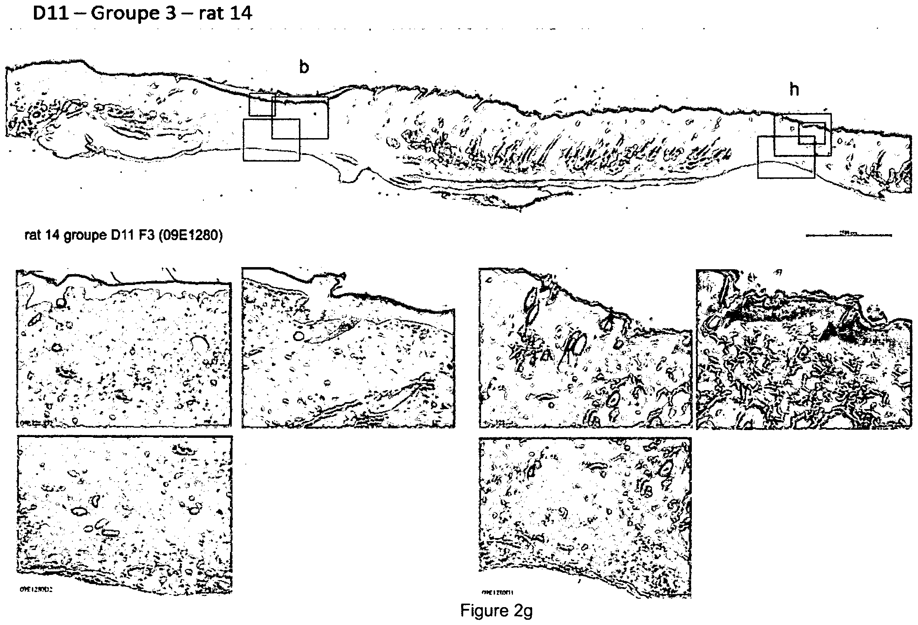

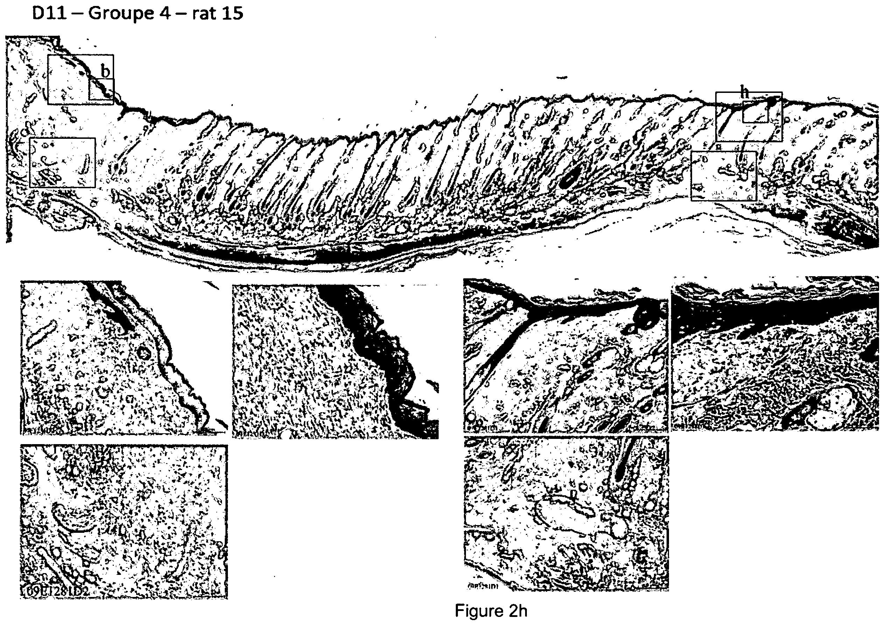

Les figures 1 et 2 représentent des résultats obtenus sur quatre groupes de rats Wistar males (250 - 300 grammes), dont chaque groupe a été traité avec un produit de composition différente.
La figure 1 correspond à l'exemple 1 et représente le taux de plaies cicatrisées dans chaque groupe en fonction du temps de guérison pour quatre groupes de rats traités avec des formulations différentes.
Les figures 2a à 2h correspondent à l'exemple 1 et sont des coupes histologiques colorées au trichrome de Masson pour chaque groupe à D7 et D11 (i.e. 7 ou 11 jours après la génération de la plaie). Sur chaque page, la barre indiquée dans les images de grande largeur correspond à 2500 µm ; les rectangles indiquent l'emplacement des images agrandies en bas de la page.
Les figures 3a et 3b correspondent à l'exemple 2 et représentent les résultats de prolifération de fibroblastes de derme humain, respectivement à D3 et D7, pour deux types d'acide hyaluronique qui se distinguent par leur masse moléculaire (20 - 50 kDa et 2 millions de Da).

### Description de l'invention

Tous les pourcentages sont des pourcentages massiques.

De manière surprenante, les inventeurs ont découvert que l'association de ces deux composants naturels dans des compositions dermatologiques induisait des effets améliorés sur la cicatrisation, ainsi que des effets anti-inflammatoires. Cet effet est lié à une certaine proportion entre ces deux composants.

La composition selon l'invention contient de 0,05% à 2,5% d'acide hyaluronique possédant une masse moléculaire ne dépassant pas 400 kDa et de 1,0% à 50% d'huile d'argan.

De préférence, la composition selon l'invention comprend 0,1 à 2,5% d'acide hyaluronique et 2,0 à 20% en poids d'huile d'argan. Encore plus préférentiellement, elle comprend 0,1 à 2,0% d'acide hyaluronique et 4,2 à 20% d'huile d'argan.

La composition selon l'invention se présente généralement sous la forme d'une crème ou pommade. Une crème est une émulsion constituée d'un mélange de composés à caractère hydrophile (phase aqueuse) et de composés à caractère hydrophobe (phase grasse). Dans le cadre de la présente invention, il s'agit d'émulsions de type « huile dans l'eau ». L'huile d'argan et les ingrédients solubles dans l'huile constituent la phase grasse et l'acide hyaluronique et les ingrédients solubles dans l'eau constituent la phase aqueuse.

L'acide hyaluronique, présente en solution aqueuse, peut être représentée, totalement ou en partie, par un sel physiologiquement acceptable, tel que son sel de sodium.

Si la proportion d'huile d'argan dans la composition est supérieure à 50%, la composition est une solution huileuse, et l'action de l'acide hyaluronique devient négligeable ; on obtient en effet les mêmes résultats qu'avec l'huile d'argan seule. Par conséquent, la composition selon l'invention ne doit pas contenir plus de 50% d'huile d'argan. Pour des concentrations en huile d'argan inférieures à 2,0%, et surtout inférieures à 1,0%, les effets sur la cicatrisation et l'inflammation sont très diminués.

D'autre part, en ce qui concerne la phase aqueuse, qui est une solution d'acide hyaluronique (et/ou d'un sel de l'acide hyaluronique) dans l'eau, lorsque la concentration d'acide hyaluronique est supérieure à 2,5%, la solution devient un gel, et la préparation d'une composition selon l'invention montrant les avantages spécifiques de l'invention n'est plus possible. Par conséquent, la composition selon l'invention ne doit pas contenir plus de 2,5% d'acide hyaluronique. Des concentrations en acide hyaluronique inférieures à 0,05% ne permettent pas d'obtenir une amélioration suffisante des propriétés cicatrisantes et anti-inflammatoires : l'effet synergétique entre les deux constituants est négligeable. Il devient appréciable à partir de 0,1%. Une composition particulièrement préférée pour ses effets dermatologiques et pour sa facilité de préparation et de conservation comporte entre 0,1% et 2,0% d'acide hyaluronique possédant une masse moléculaire ne dépassant pas 400 kDa et entre 4,2 et 20% d'huile d'argan.

L'acide hyaluronique et l'huile d'argan utilisés dans la composition selon l'invention peuvent être obtenus par toute technique connue de l'homme du métier.

De préférence, on utilise dans la composition selon l'invention une huile d'argan pure, c'est-à-dire une huile d'argan qui a été préparée directement par pression et qui ne comporte aucun additif, tels que des protéines ou vitamines. Avantageusement, elle présente un indice de réfraction compris entre 1,4687 et 1,4689 (de préférence d'environ 1,4688), mesurée dans des conditions de température normales ; de préférence elle présente un indice d'acide d'environ 0,1 et un indice de peroxyde d'environ 0,4. Avantageusement, son taux insaponifiable ne dépasse pas la valeur de 1.

De préférence, l'huile d'argan utilisée dans la composition selon la présente invention contient entre 80 et 84% d'acides gras insaturés dont typiquement entre 45 et 46% d'acide oléique, entre 32,5 et 33,5% d'acide linoléique, d'autres acides gras tels que acide palmitoléïque, acide linolénique, acide gadoléïque chacun en une quantité inférieure à 1%. Avantageusement, l'huile d'argan utilisable dans la composition selon la présente invention contient également environ 20% d'acides gras saturés dont environ 13% d'acide palmitique, 5,0 à 5,5% d'acide stéarique, d'autres acides gras tels que acide myristique, acide pentadécanoïque, acide margarique, acide arachidique, chacun en une quantité inférieure à 0,1%.

De préférence, l'huile d'argan utilisée dans la composition selon la présente invention contient également environ 600 à 650 mg/kg de tocophérols, dont environ 400 à 450 mg/kg de vitamine E (α-tocophérol). L'huile d'argan utilisée dans la composition selon la présente invention contient également des stérols (spinastérol et schotténol, ce dernier représentant avantageusement environ la moitié (en masse) des stérols présents, et de préférence compris entre 43% et 51%). L'acide hyaluronique utilisé dans la composition de la présente invention est un acide hydrolysé qui présente une masse moléculaire ne dépassant pas 400 kDa, de préférence comprise entre 10 et 300 kDa, et encore plus préférentiellement entre 10 et 100 kDa. Il a en effet été constaté que l'acide hyaluronique dont la masse moléculaire est comprise entre ces deux valeurs a un effet optimal sur la croissance de fibroblastes de derme humain. En effet, l'effet cicatrisant est obtenu plus rapidement avec l'acide hyaluronique de faible masse molaire, ainsi que le démontre les études de prolifération de fibroblastes humains présentés dans l'exemple 2 ci-après. Les acides hyaluroniques à grande masse moléculaire, supérieure à 400 kDa, et en particulier les acides hyaluroniques non hydrolysés (natives) tels que ceux utilisés en orthopédie pour la viscosupplémentation, ne semblent pas avoir d'effet synergétique avec l'huile d'argan. Le meilleur résultat a été obtenu avec une masse moléculaire comprise entre 20 et 50 kDa.

La masse moléculaire de l'acide hyaluronique peut être déterminée selon la pharmacopée européenne à partir des viscosités intrinsèques mesurées à l'aide d'un viscosimètre Ubbelhode à 25°C pour des solutions à différentes concentrations d'acide.

Dans un autre mode de réalisation, la composition selon l'invention peut comprendre en outre comme ingrédient de là glycérine (propan-1,2,3-triol). La glycérine, ou glycérol, ou propan-1,2,3-triol est classiquement utilisée comme agent hydratant, solvant et lubrifiant dans les produits cosmétiques.

Dans encore un autre mode de réalisation, la composition selon l'invention peut comprendre en outre comme ingrédient du D-Panthénol ou dexpanthénol ou provitamine B5. Le D-Panthénol est un anti-inflammatoire et un puissant hydratant qui redonne souplesse et élasticité à la peau. En raison de ses propriétés, il est communément utilisé dans les préparations cosmétiques pour la peau.

Dans encore un autre mode de réalisation, la composition selon l'invention peut comprendre en outre comme ingrédient de l'huile essentielle de romarin. L'huile essentielle de romarin est utilisée classiquement pour soigner les blessures (effet antiseptique léger).

La composition selon la présente invention peut être utilisée avantageusement pour le traitement ou la prévention des plaies et irritations cutanées chez les mammifères. La cicatrisation d'une plaie est un phénomène biologique naturel : les tissus des mammifères sont capables de réparer des lésions localisées. Une plaie induit dans l'organisme des processus biologiques et active le système vasculaire et le tissu conjonctif. Cela aboutit à une cicatrisation avec ré-épithélialisation du tissu de remplacement. On distingue classiquement la cicatrisation primaire, ou de première intention et la cicatrisation secondaire, ou de seconde intention. Généralement, la cicatrisation de première intention se produit pour une plaie réalisée par un objet tranchant, et par conséquent à bords nets et bien apposés, en l'absence d'infection. La cicatrisation de seconde intention concerne les plaies avec perte de substance et/ou infection, qui empêchent la réunion des bords de la plaie. La fermeture de la plaie est dans cas obtenue par production de nouveaux tissus, i.e. les tissus de granulation.

La cicatrisation d'une plaie comporte trois phases successives : la phase exsudative, la phase proliférative avec développement du tissu de granulation, la phase de différenciation avec maturation cellulaire, développement de la cicatrice et épithélialisation. Dans la phase exsudative, des phénomènes inflammatoires se produisent, immédiatement après le traumatisme débutent des sécrétions à partir de vaisseaux sanguins et lymphatiques. La coagulation est induite par activation de la thrombokinase qui est libérée et il en résulte la formation de fibrine. L'augmentation de la perméabilité capillaire favorise le passage de plasma sanguin avec anticorps, leucocytes et macrophages vers la région traumatisée. Les tissus nécrosés, les corps étrangers et les microbes sont éliminés et détruits.

Quelques jours après la blessure, l'organisme commence à produire du tissu de granulation. Les fibroblastes produisent en premier lieu des mucopolysaccarides qui serviront de matrice à l'élaboration des fibres collagènes du tissu conjonctif. La synthèse du collagène se fait dans les fibroblastes. Les fibres collagènes, au début isolées, se renforcent et s'assemblent en faisceaux. La fibre collagène définitive et insoluble se constitue, et est incorporée dans la matrice mucopolysaccharide. Simultanément, de nouveaux vaisseaux sanguins, les néocapillaires se développent dans cette matrice pour assurer la nutrition du tissu nouvellement formé. Dans les plaies les plus importantes, les néocapillaires se présentent à la surface de la plaie avec un aspect de granulation rouge vif.

Entre le sixième et le dixième jour en moyenne, débute la phase de différenciation avec maturation cellulaire, développement de la cicatrice et épithélialisation. La maturation des fibres collagènes commence. La plaie se rétracte sous l'influence de cellules particulières, les myofibroblastes. Le tissu de granulation devient progressivement plus ferme, il s'appauvrit en eau et en contient de moins en moins de vaisseaux. Il se transforme en tissu cicatriciel qui, à son tour, favorisera la rétraction cicatricielle. L'épithélialisation marque la fin de la cicatrisation.

Les inventeurs ont trouvé que la formulation selon l'invention a un effet positif sur la néovascularisation et sur la maturation du tissu de granulation, ainsi que le montre l'exemple 1 ci-après.

Les inventeurs ont trouvé que la composition selon la présente invention peut être utilisée avec bénéfice chez les mammifères, par exemple chez l'être humain, chez les espèces équines, canines, félines, caprins, ovines, bovins. La composition selon l'invention est particulièrement bien adaptée au traitement et à la prévention des plaies chez le cheval. On sait que la guérison cutanée est souvent problématique chez le cheval. La cicatrisation des plaies est souvent de mauvaise qualité chez ce mammifère. La cicatrisation chez cheval est de mauvaise qualité pour deux raisons : La phase d'inflammation est courte, la phase de granulation est excessive, du à un désordre fibrino-prolifération. Cela est décrit en détail dans les publications suivantes : Jacobs K.A., Leach D.H., Fretz P.B., Townsend H.G.G. :"Comparative aspects of the healing of excisional wounds on the leg and body of horses", Vet. Surg. 1984;13:83-90 ; Knottenbelt D.C., "Equine wound management: are there significant differences in healing at different sites on the body ?" : Vet. Dermatol. 1997; 8: 273-90. On peut noter que le même phénomène peut exister chez l'humain, on parle dans ce cas de cicatrice kéloïde.

Les effets de la composition selon l'invention seront mieux compris en référence aux exemples décrits ci-après qui illustrent quelques applications.

### Exemples

Pour la préparation de la composition selon l'invention, on a utilisé une huile d'argan issue de l'agriculture biologique commercialisée par la société ActiBio. Elle présentait un indice de réfraction de 1,4688, un indice de peroxyde de 0,4 , un indice d'acide de 0,1 et comportait 18% d'acides gras saturés et 82% d'acides gras insaturés. Elle comportait 45,7 d'acide oléique, 33,2% acide linoléique, 13,1 d'acide palmitique et 5,3% d'acide stéarique ; parmi les stérols elle comportait 39,7% spinastéol et 47,1% schottenol.

Sauf indication contraire, on a utilisé un acide hyaluronique hydrolysée (n° CAS 9004-61-9) présentant une distribution de masse moléculaire dans l'intervalle 20-50 KDa, commercialisé par la société Soliance sous la marque Primalhydral 50 ™ sous la forme d'une poudre ; cette poudre a été dissoute dans une phase aqueuse.

### Exemple 1 : Préparation d'un crème et étude de la cicatrisation de plaies chez le rat

L'objectif de cette étude était de démontrer l'effet cicatrisant d'une formulation selon l'invention type crème, prévue pour une utilisation en tant que crème cicatrisante pour le traitement topique de la peau.

La crème a été formulée en associant d'acide hyaluronique avec l'huile d'argan dans les quantités suivantes : 0,1% / 5% (pourcent massiques), de la manière suivante :.

La phase huileuse (un mélange de huile d'argan, de glycérine et de glycérides et triglycérides d'origine naturelle) a été introduite dans un fondoir chauffé à 70-75°C. La solution aqueuse (comportant de l'eau et divers alcools hydrophiles) a été chauffée à 70 - 75°C, et la phase grasse a été incorporée dans la phase aqueuse sous agitation vigoureuse (agitateur planétaire). Les deux phases étant à la même température lorsqu'elles rentrent en contact l'une avec l'autre, l'émulsion commence à prendre rapidement avec l'ajout de l'émulsifiant. Le mélange a été maintenu en agitation (100 tr/min) pendant quelques minutes puis refroidi à la température ambiante 25 °C, toujours sous agitation. Ensuite, on a introduit des produits sensibles à la température : une solution aqueuse d'acide hyaluronique, du D-panthenol et de l'huile essentielle de romarin ; ces ingrédients ont été rajoutés les uns après les autres. Le mélange a été maintenu en agitation et une phase liquide contenant les conservateurs a été rajoutée en dernier.

La composition totale était (pourcentages massiques par rapport la masse totale) :
- Phase aqueuse 35 % (dont 0,10 % halyuronate de sodium),
- phase grasse 53 % (dont 5,0 % huile d'argan),
- émulsifiants (parmi lesquels : PEG, glycéryl stéarate, arachidyl glycoside), stabilisateurs d'émulsion et agents de contrôle de viscosité (parmi lesquels : sodium acrylate, alcool arachidique) 11 %,
- conservateurs 1%.

L'étude de cicatrisation a été réalisée sur un modèle de plaies dermo-épidermiques (punch de 8 mm de diamètre) chez le rat Wistar en testant les 4 formulations suivantes :
- Le groupe 1 (G1) a reçu une solution d'acide hyaluronique (Formulation 1).
- Le groupe 2 (G2, groupe témoin) a reçu du sérum physiologique (Formulation 2).
- Le groupe 3 (G3) a reçu la composition selon l'invention (Formulation 3).
- Le groupe 4 (G4) a reçu de l'huile d'argan (Formulation 4).

Afin de démontrer l'effet de l'association de l'acide hyaluronique et de l'huile d'argan, les quantités respectives d'acide hyaluronique et d'huile d'argan utilisées dans la formulation selon l'invention (formulation 3) ont été testées séparément pour apprécier l'effet de l'addition de ces deux composés (formulation 1 pour l'acide hyaluronique et formulation 4 pour l'huile d'argan). Le groupe témoin (G2) a été traité avec du sérum physiologique (formulation 2).

Les compositions des différentes formulations testées sont les suivantes :
- Formulation 1 solution d'acide hyaluronique à 0,1% dans du sérum physiologique,
- Formulation 2 : sérum physiologique pur,
- Formulation 3 : formulation galénique contenant 0,1% d'acide hyaluronique et 5% d'huile d'argan,
- Formulation 4 : huile d'argan pure.

Afin de vérifier l'effet de la composition selon l'invention, les paramètres suivants ont été étudiés :
- la vitesse et la qualité de la cicatrisation chez le rat,
- Le développement du tissu de granulation, représenté sur les figures 2a à 2h,
- L'angiogenèse, tel que représenté sur les figures 2a à 2h.
Chaque animal a été soumis au test suivant :
- Génération des plaies : 2 punchs (réalisés à l'emporte pièce) paravertébraux de 8 mm de diamètre sur chaque flanc de l'animal.
- Suivi de la cicatrisation :
   ∘ Renouvellement des pansements : tous les 2 jours.
   ∘ lectures et photos à chaque renouvellement.
- Traitement : Application des différentes formulations testées à D0, D2 et D4 (où Dx indique le x-ième jour qui suit la génération de la plaie).
- Analyse hiétopathologique :
   ∘ Prélèvement des sites.
   ∘ Fixation et inclusion en paraffine.
   ∘ Réalisation d'une lame histologique par site (soit un total de 32 lames).
   On entend par groupe 1 ou G1 le groupe de rats ayant reçu la formulation 1. On entend par groupe 2 ou G2 le groupe de rats ayant reçu la formulation 2.
   On entend par groupe 3 ou G3 le groupe de rats ayant reçu la formulation 3.
   On entend par groupe 4 ou G4 le groupe de rats ayant reçu la formulation 4.

### Résultats

Les résultats sont illustrés dans les figures 1 et 2.

### Planimétrie et plaies fermées par groupes

Des photographies macroscopiques ont été analysées avec un logiciel d'image (Image J64) qui permet de mesurer la surface non cicatrisée de chaque plaie pour établir la planimétrie et la vitesse de cicatrisation en fonction du temps.

La courbe de planimétrie des différents groupes représentée sur la figure 1 montre une différence significative entre les groupes 1 (acide hyaluronique), 3 (formulation selon l'invention) et 4 (huile d'argan) vis-à-vis du groupe 2 témoin (sérum physiologique).

A D9 (i.e. au 9^{ème} jour après la génération de la plaie), les 4 groupes se confondent et peu de différence est observée, la surface de plaie résiduelle est exprimée en %.

Pour distinguer les groupes entre eux, l'analyse du nombre de plaies fermées par formulation, est une analyse plus fine, qui permet de distinguer les trois groupes entre eux (1, 3 et 4).

A partir de D7, le groupe 3 (formulation selon l'invention) se distingue nettement du groupe 4 (huile d'Argan seule) et présente un effet légèrement supérieur au groupe 1 (acide hyaluronique). Il en est de même à D9.

A D11, les groupes 1 et 3 présentent le même nombre de plaies fermées.

A D14, seules quelques plaies du groupe témoin ne sont pas cicatrisées.

Au niveau macroscopique, la formulation selon l'invention présente donc un effet sur la fermeture des plaies supérieur à celui de l'acide hyaluronique seul et de l'huile d'Argan seule.

### Analyse histologique

Pour apprécier plus finement la qualité de la cicatrisation, la coloration au trichrome selon Masson (coloration du collagène au Vert Lumière ou Bleu d'aniline, associé à une coloration nucléaire par l'hématoxyline, et une coloration cytoplasmique par un mélange de colorants acides - fuchsine Ponceau) a été réalisée. Ce procédé permet d'apprécier les paramètres suivants : tissu de granulation, néovascularisation et collagène. Le barème d'appréciation utilisé est le suivant :
- Synthèse et maturité du collagène : échelle de 1 à 4 selon l'intensité de la couleur (de plus en plus turquoise, représenté par niveau de gris sur les figures).
- Néo vaisseaux : comptage du nombre de petits vaisseaux par unité de surface.
- Annexes : comptage du nombre de bulbes pileux par unité de surface.

Les résultats obtenus à D7 et D11 pour chaque groupe sont présentés dans les tableaux ci-dessous. Ces résultats sont également illustrés par les figures 2a à 2h.

**Tableau 1**

| Résultats à D7 | G1 | G2 | G3 | G4 |
|---|---|---|---|---|
| Collagène | 2 | 1 | 3 | 2 |
| Néovaisseaux /mm² | 15 | 7 | 18 | 15 |
| Annexes (poils) /mm² | 3 | 2 | 3 | 1 |

**Tableau 2**

| Résultats à D11 | G1 | G2 | G3 | G4 |
|---|---|---|---|---|
| Collagène | 3 | 2 | 4 | 3 |
| Epaisseur TG | ++ | + | ++ | ++ |
| Annexes/mm² | 2 | 5 | 6 | 3 |

Dans ces tableaux, les abréviations et symboles suivants sont utilisés :
- TG :: Tissu de granulation.
- + :: Epaisseur du TG inférieure à l'épaisseur du derme non lésé (non comblée).
- ++ :: Epaisseur du tissu de granulation identique au tissu normal (comblée).

Les tableaux 1 et 2 montrent la supériorité de la quantité de néovaisseaux formés et la supériorité de la maturation du collagène lors de l'utilisation de la composition selon l'invention (formulation 3) pour le traitement des plaies chez le rat Wistar, comparativement aux autres formulations : sérum physiologique (formulation 2) et produits utilisés seuls (acide hyaluronique (formulation 1) et huile d'argan (formulation 4)) à la même concentration que celle de la formulation selon l'invention.

La formulation selon l'invention possède donc une action significativement supérieure à l'acide hyaluronique seul et à l'huile d'argan seule sur la vitesse et la qualité de la cicatrisation des plaies.

### Exemple 2 : étude de l'influence de la mase moléculaire de l'acide hyaluronique sur la culture de fibroblastes de derme humain

### 1. Solutions mères

La solution mère des différents polymères d'acide hyaluronique est préparée à une concentration d'acide hyaluronique de 2 mg/mL dans du milieu DMEM (milieu Eagle modifié de Dulbecco, un milieu de culture cellulaire qui contient des acides aminés, des sels minéraux (KCI, MgSO₄, NaCl, NaH₂PO₃), du glucose et des vitamines (acide folique, nicotinamide, riboflavine et vitamine B12) et les dilutions sont ensuite réalisées en milieu DMEM + 2,5% SVF (sérum de veau foetal) + antibiotique. On a utilisé un acide hyaluronique présentant une masse moléculaire comprise entre 20 et 50 kDa, et un acide hyaluronique à forte masse moléculaire de l'ordre de 2 millions de Da.

### 2. Culture

La souche utilisée est: M228 F01, fibroblastes dermiques humains.

Les fibroblastes utilisés sont ensemencés à raison de 5x10³ cellules/puits dans des plaques 96 puits (passage 7 pour l'essai n°1, passage 9 pour l'essai n°2) en présence de 200 µl de milieu de culture complet (DMEM + 10% SVF + antibiotiques). 24h après l'ensemencement, les cellules sont sevrées dans du milieu DMEM sans sérum pendant 4 heures. Puis à D0, les différentes dilutions de chaque produit sont additionnées, avec 3 puits par condition de culture.

3 témoins sont aussi ajoutés (3 puits/témoin) :
- DMEM sans sérum,
- milieu appauvri en sérum (DMEM + 2,5% SVF + antibiotique)
- milieu complet (DMEM + 10% SVF + antibiotique).

Le milieu est renouvelé à D3 pour les cultures étudiées à D7.

### 3. Quantification de la prolifération

La prolifération des cellules est évaluée par un test colorimétrique, dit test MTT, d'une part après 3 jours et d'autre part après 7 jours de culture. Ce test est basé sur le principe d'une réduction du sel jaune de tétrazolium (MTT) (bromure de 3-(4,5-dimethylthiazol-2-yl)-2,5-diphenyl tetrazolium) [en un produit formazan bleu-violet par les réductases NADPH mitochondriales des cellules vivantes.

### 4. Test MTT

La prolifération des cellules est évaluée par le test colorimétrique : lorsque le sel de tétrazolium (MTT) 5mg/ml en PBS) est ajouté au milieu de culture, un produit formazan bleu-violet est formé par les réductases NADPH mitochondriales des cellules vivantes. Les cristaux sont ensuite dissous par un mélange éthanol 100° - DMSO (v/v). La coloration du surnageant, proportionnelle à la quantité de cellules vivantes, est mesurée par lecture de la densité optique (D.O) au spectrophotomètre à une longueur d'onde de 570 nm.

Le test est effectué à D3 et à D7 (D0 correspondant à l'addition des solutions à tester).

### 5. Analyse des résultats

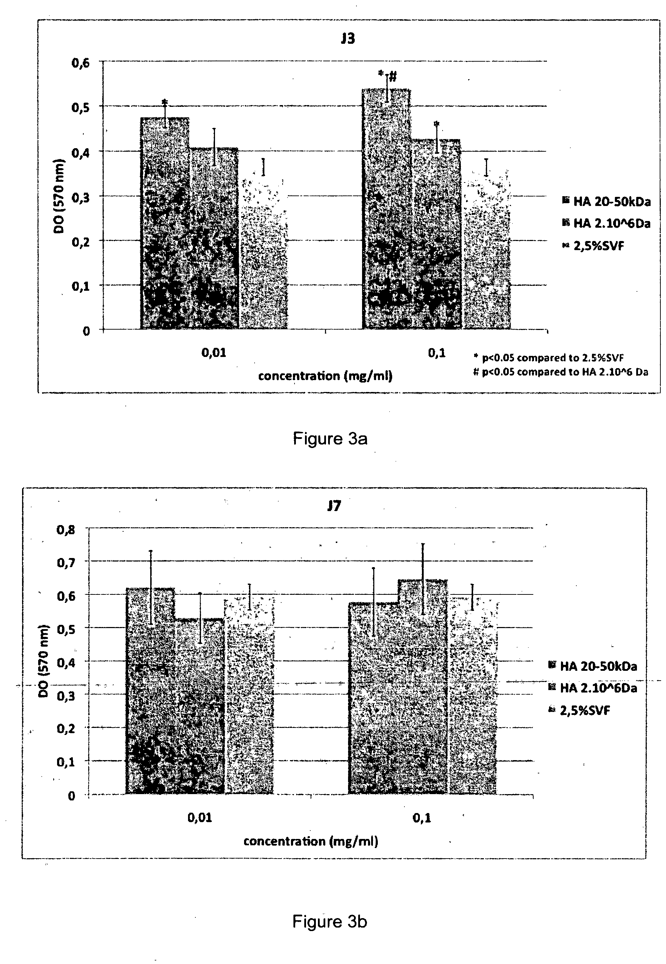

Les moyennes des densités optiques des 3 puits/échantillon sont représentées dans des graphes sur les figures 3a et 3b. L'évaluation statistique est réalisée par le test *t* de Student. La différence, par rapport au témoin DMEM + 2.5% SVF, est considérée comme significative pour p 0,05.

Ainsi, la culture de fibroblastes de derme humain en présence d'acide hyaluronique de différentes masse molaire montre que l'acide hyaluronique de faible masse moléculaire est plus actif sur la culture de fibroblastes de derme humain à D3 que l'acide hyaluronique de plus haute masse molaire. Le test est statistiquement significatif par rapport au témoin (SVF à 2,5%) et par rapport à l'acide hyaluronique de forte masse moléculaire et ce à deux concentrations (0,01 et 0,1 mg/ml).

En revanche, on constate que l'effet prolifératif des deux acides hyaluroniques n'est plus statistiquement différent à D7 et ce quelle que soit la concentration en acide hyaluronique.

Cet essai permet de comprendre pourquoi la composition selon la présente invention comprend de préférence un acide hyaluronique de faible masse molaire (typiquement entre 20 et 50 kDa) qui donne de bien meilleurs résultats de prolifération de fibroblastes de derme humain dans un temps court (D3).

### Exemple 3: Résultats qualitatifs des applications de la crème cicatrisante aux chevaux

Plusieurs essais ont été réalisés avec la crème selon l'exemple 1 (formulation 3) sur chevaux de selle et les applications ont concerné le traitement des blessures dans les parties suivantes : blessures au garrot, passage de sangles, commissures des lèvres.

### a) Blessures au garrot :

Le garrot chez le cheval est une zone particulièrement sensible qui se trouve à la base de l'encolure. De par sa situation, à la jonction de l'encolure et du dos, cette zone est très exposée, car c'est sur cette zone que repose la plus grande partie du poids du cavalier. Les plaies à cet endroit sont fréquentes et peuvent rapidement dégénérer en blessures graves si le cheval n'est pas soigné. La cause majoritaire de ces blessures est une selle mal dégarrotée et/ou une couverture mal ajustée.

La crème selon l'invention a été appliquée dans deux types de blessures au garrot chez des chevaux de clubs équestres.
1) Un harnachement mal entretenu et posé avec un tapis de selle mal ajusté.
2) Une blessure due à une peau mal nettoyée avec des poussières et des grains de sables qui frottent entre la selle et la peau.

La crème a été utilisée, une fois par jour pendant 3 jours après nettoyage à chaque fois des blessures au sérum physiologique. La crème a laissé un film hydroprotecteur qui a pour conséquence d'éviter à la blessure de sécher et de stimuler la réparation.

Au bout de deux jours d'application de crème, la blessure a diminué pour disparaître au bout de 7 jours et les poils ont repris leur couleur d'origine.

### b) Passage de sangle:

Le passage de sangle est une zone derrière les antérieurs qui permet de maintenir la selle. C'est une zone ou le harnachement est responsable des blessures qui peuvent rendre « inmontable » le cheval pendant plusieurs semaines.

Les causes majeurs de blessures dans cette partie du cheval sont dues à :
1) Une sangle mal nettoyée qui va frotter sur la peau et finir par l'irriter et provoquer une plaie.
2) Une sangle mal adaptée va avoir le même effet.

La crème a été utilisée, une fois par jour pendant 3 jours après nettoyage à chaque fois de la blessure au sérum physiologique.

Les blessures ont disparues au bout d'une semaine de traitement journalier, après nettoyage de la zone à traiter au sérum physiologique avant chaque application.

### c) Commissure des lèvres:

Les parties du filet sont à l'origine d'atteintes qui peuvent être provoquées par :
1) La gourmette de la bride ou du pelham trop serrée qui peut engendrer une plaie sous le menton.
2) Un mors mal adapté qui peut blesser le cheval à la commissure des lèvres.
3) Le filet qui peut blesser le front ou les côtés de la tête du cheval.

Sur un cas de blessures à la commissure des lèvres, la crème a été appliquée une fois par jour pendant 4 jours et a montré des résultats spectaculaires de cicatrisation.

## Revendications

1. Composition, notamment sous la forme d'une crème, pommade, baume ou d'un gel, pour le traitement topique de la peau, **caractérisée en ce qu'**elle comprend, dans un milieu physiologiquement acceptable :
- 1,0 à 50 % massiques d'huile d'argan, et
- 0,05 à 2,5 % massiques d'acide hyaluronique, ce dernier étant présent éventuellement sous la forme d'un sel physiologiquement acceptable, et possédant une masse moléculaire ne dépassant pas 400 kDa,

2. Composition selon la revendication 1, **caractérisée en ce que** l'acide hyaluronique possède une masse moléculaire comprise entre 10 et 300 kDa.

3. Composition selon la revendication 2, **caractérisée en ce que** l'acide hyaluronique possède une masse moléculaire comprise entre 10 et 100 kDa.

4. Composition selon l'une des revendications 3, **caractérisée en ce que** l'acide hyaluronique possède une masse moléculaire comprise entre 20 et 50 kDa.

5. Composition selon l'une des revendications 1 à 4, **caractérisée en ce qu'**elle comprend ;
- 2,0 à 50% massiques d'huile d'argan, et
- 0,1% à 2,5% d'acide hyaluronique.

6. Composition selon la revendication 5, **caractérisée en ce qu'**elle comprend :
- 4,2 à 20% massiques d'huile d'argan, et
- 0,1% à 2,0% d'acide hyaluronique.

7. Composition selon l'une quelconque des revendications 1 à 6, **caractérisée en ce qu'**elle contient, en tant que seuls principes actifs, l'huile d'argan et l'acide hyaluronique, ce dernier éventuellement sous la forme d'un sel physiologiquement acceptable, dans les proportions indiquées.

8. Composition selon l'une des revendications 1 à 7, **caractérisée en ce qu'**elle comprend en plus un ou plusieurs principes actifs sélectionné dans le groupe constitué par les antibiotiques, les antiseptiques, les antimycosiques, les anti-inflammatoires, les antalgiques à application topique, les antalgiques à effet systémique.

9. Composition selon l'une quelconque des revendications 1 à 8, **caractérisée en ce que** l'huile d'argan est une huile pure, de préférence avec un indice de réfraction compris entre 1,4687 et 1,4689.

10. Composition selon l'une quelconque des revendications 1 à 9 **caractérisée en ce qu'**elle comprend en outre un ou plusieurs des ingrédients suivants : glycérine, huile de romarin, D-Panthenol.

11. Procédé pour la fabrication d'une telle composition selon l'une quelconque des revendication 1 à 10 sous la forme d'une émulsion de type « huile dans l'eau », dans lequel on prépare d'abord, à une température supérieure à 45°C et sous agitation, une émulsion de type « huile dans l'eau », ladite phase huileuse comprenant l'huile d'argan, puis on laisse refroidir, de préférence à une température ne dépassant pas 30°C et de préférence ne dépassant pas 25°C, et on ajoute à ladite émulsion une phase aqueuse comprenant l'acide hyaluronique ou un de ses sels physiologiquement acceptables.

12. Composition selon l'une quelconque des revendications 1 à 10 pour son utilisation dans le traitement de plaies cutanées ou de l'irritation cutanée chez les mammifères, et de préférence chez l'homme ou chez le cheval.

## Patentansprüche

1. Zusammensetzung, insbesondere in der Form einer Creme, Salbe, eines Balsams oder eines Gels, für die topische Behandlung der Haut, **dadurch gekennzeichnet, dass** sie in einem physiologisch annehmbaren Medium Folgendes umfasst:
- 1,0 bis 50 Massen-% Arganöl, und
- 0,05 bis 2,5 Massen-% Hyaluronsäure, wobei die Letztere gegebenenfalls in der Form eines physiologisch annehmbaren Salzes vorliegt und ein Molekulargewicht besitzt, das 400 kDa nicht überschreitet.

2. Zusammensetzung nach Anspruch 1, **dadurch gekennzeichnet, dass** die Hyaluronsäure ein Molekulargewicht im Bereich von 10 und 300 kDa besitzt.

3. Zusammensetzung nach Anspruch 2, **dadurch gekennzeichnet, dass** die Hyaluronsäure ein Molekulargewicht im Bereich von 10 und 100 kDa besitzt.

4. Zusammensetzung nach einem der Ansprüche 3, **dadurch gekennzeichnet, dass** die Hyaluronsäure ein Molekulargewicht im Bereich von 20 und 50 kDa besitzt.

5. Zusammensetzung nach einem der Ansprüche 1 bis 4, **dadurch gekennzeichnet, dass** sie Folgendes umfasst:
- 2,0 bis 50 Massen-% Arganöl, und
- 0,1 % bis 2,5 % Hyaluronsäure.

6. Zusammensetzung nach Anspruch 5, **dadurch gekennzeichnet, dass** sie Folgendes umfasst:
- 4,2 bis 20 Massen-% Arganöl, und
- 0,1 % bis 2,0 % Hyaluronsäure.

7. Zusammensetzung nach einem der Ansprüche 1 bis 6, **dadurch gekennzeichnet, dass** sie als einzige Wirkstoffe Arganöl und Hyaluronsäure enthält, gegebenenfalls in der Form eines physiologisch annehmbaren Salzes in den angegebenen Anteilen.

8. Zusammensetzung nach einem der Ansprüche 1 bis 7, **dadurch gekennzeichnet, dass** sie zusätzlich einen oder mehrere Wirkstoffe umfasst, ausgewählt aus der Gruppe, bestehend aus Antibiotika, Antiseptika, Antimykotika, Antiphlogistika, Analgetika zur topischen Anwendung, Analgetika mit systemischer Wirkung.

9. Zusammensetzung nach einem der Ansprüche 1 bis 8, **dadurch gekennzeichnet, dass** das Arganöl ein reines Öl ist, vorzugsweise mit einem Brechungsindex im Bereich von 1,4687 und 1,4689.

10. Zusammensetzung nach einem der Ansprüche 1 bis 9, **dadurch gekennzeichnet, dass** sie des Weiteren einen oder mehrere der folgenden Inhaltsstoffe umfasst: Glycerin, Rosmarinöl, D-Panthenol.

11. Verfahren zur Herstellung einer solchen Zusammensetzung nach einem der Ansprüche 1 bis 10 in der Form einer Emulsion des Typs "Öl-in-Wasser", wobei man zuerst, bei einer Temperatur über 45 °C und unter Rühren, eine Emulsion des Typs "Öl-in-Wasser" zubereitet, wobei die ölige Phase das Arganöl umfasst, man anschließend abkühlen lässt, vorzugsweise auf eine Temperatur, die 30 °C nicht überschreitet und vorzugsweise 25 °C nicht überschreitet, und man zu der Emulsion eine wässrige Phase, umfassend die Hyaluronsäure oder eines ihrer physiologisch annehmbaren Salze, hinzugibt.

12. Zusammensetzung nach einem der Ansprüche 1 bis 10 zur Verwendung bei der Behandlung von Hautwunden oder Hautreizungen bei Säugetieren, und vorzugsweise beim Menschen oder beim Pferd.

## Claims

1. Composition, particularly in the form of a cream, ointment, balm or a gel, for topical skin treatment, **characterised in that** it comprises, in a physiologically acceptable medium:
- 1.0% to 50% by mass of argan oil, and
- 0.05% to 2.5% by mass of hyaluronic acid, the latter being present optionally in the form of a physiologically acceptable salt, and having a molecular mass not exceeding 400 kDa.

2. Composition according to claim 1, **characterised in that** the hyaluronic acid has a molecular mass between 10 and 300 kDa.

3. Composition according to claim 2, **characterised in that** the hyaluronic acid has a molecular mass between 10 and 100 kDa.

4. Composition according to one of claims 3, **characterised in that** hyaluronic acid has a molecular mass between 20 and 50 kDa.

5. Composition according to one of claims 1 to 4, **characterised in that** it comprises:
- 2.0% to 50% by mass of argan oil, and
- 0.1% to 2.5% by mass of hyaluronic acid.

6. Composition according to claim 5, **characterised in that** it comprises:
- 4.2% to 20% by mass of argan oil, and
- 0.1% to 2.0% by mass of hyaluronic acid.

7. Composition according to any one of claims 1 to 6, **characterised in that** it contains, as sole active substances, argan oil and hyaluronic acid, the latter optionally in the form of a physiologically acceptable, salt, in the stated proportions.

8. Composition according to one of claims 1 to 7, **characterised in that** it further comprises one or a plurality of active substances selected in the group consisting of antibiotics, antiseptics, antimycotics, anti-inflammatories, analgesics having topical application, analgesics having a systemic effect.

9. Composition according to any one of claims 1 to 8, **characterised in that** the argan oil is a pure oil, preferably with a refractive index between 1.4687 and 1.4689.

10. Composition according to any one of claims 1 to 9 **characterised in that** it further comprises one or a plurality of the following ingredients: glycerin, rosemary oil, D-Panthenol.

11. Method for the manufacture of such a composition according to any one of claims 1 to 10 in the form of an "oil-in-water" type emulsion, wherein, at a temperature greater than 45°C and under stirring, an "oil-in-water" type emulsion is first prepared, said oil phase comprising argan oil, then allowed to cool, preferably to a temperature not exceeding 30°C and preferably not exceeding 25°C, and an aqueous phase comprising hyaluronic acid or one of the physiologically acceptable salts thereof is added.

12. Composition according to any one of claims 1 to 10 for use thereof in the treatment of skin wounds or of skin irritation in mammals, and preferably in humans or in horses.
